# FASCICULE DE BREVET EUROPEEN

(11) **EP 1 414 447 B1**
(45) Date de publication et mention de la délivrance du brevet: **26.07.2006**
(21) Numéro de dépôt: 02774830.0
(22) Date de dépôt: 25.07.2002
(51) Int. Cl.: A61K 31/426, A61K 31/427, A61P 43/00, A61P 27/02

(54) **UTILISATION DE DERIVES DE THIAZOLES POUR PREPARER UN MEDICAMENT DESTINE A PROTEGER LES MITHOCHONDRIES**
VERWENDUNG VON THIAZOL-DERIVATIVEN ZUR HERSTELLUNG EINES MEDIKAMENTS ZUM SCHUTZ DER MITOCHONDRIEN
USE OF THIAZOLE DERIVATIVES FOR PREPARING A MEDICINE FOR PROTECTING MITOCHONDRIA

(30) Priorité: 26.07.2001 FR 0109979
(43) Date de publication de la demande: 06.05.2004
(73) Titulaire: SOCIETE DE CONSEILS DE RECHERCHES ET D'APPLICATIONS SCIENTIFIQUES (S.C.R.A.S.), 75016 Paris (FR)
(72) Inventeur: AUGUET, Michel, F-91120 Palaiseau (FR); CHABRIER DE LASSAUNIERE, Pierre-Etienne, F-75016 Paris (FR); HARNETT, Jeremiah, F-91190 Gif-sur-Yvette (FR)
(74) Mandataire: Bourgouin, André
(86) Numéro de dépôt international: PCT/FR2002/002660
(87) Numéro de publication internationale: WO 2003/009843

(56) Documents cités:
- EP-A- 0 397 365
- WO-A-00/54729
- WO-A-00/76971
- WO-A-01/26656
- FR-A- 2 764 889
- FR-A- 2 800 615
- US-A- 5 364 862
- US-B1- 6 262 069
- WYATT, P. G. ET AL: "Structure-activity relationship investigations of a potent and selective benzodiazepine oxytocin antagonist" BIOORGANIC & MEDICINAL CHEMISTRY LETTERS (2001), 11(10), 1301-1305, XP001066283

## Description

La présente invention a pour objet l'utilisation des dérivés de thiazoles de formule générale **(I)** décrite plus loin pour préparer un médicament destiné à protéger les mitochondries, et en particulier un médicament destiné à prévenir ou à traiter la cirrhose du foie.

La demanderesse avait déjà décrit dans la demande de brevet PCT WO 01/26656 des dérivés de thiazoles, d'oxazoles et d'imídazoles inhibant la peroxydation lipidique et/ou les monoamine oxydases et/ou modulant les canaux sodiques. Ces propriétés confèrent à ces composés des applications thérapeutiques intéressantes comme, notamment, le traitement de maladies neurodégénératives ou de la douleur.

La demanderesse vient à présent de découvrir de façon surprenante que certains composés particuliers parmi ceux décrits dans la demande de brevet PCT WO 01/26656 possédaient en outre la faculté de protéger les mitochondries, ce qui leur ouvre de nouvelles applications thérapeutiques comme par exemple la prévention ou le traitement de la cirrhose du foie.

En effet, ces composés s'opposent au gonflement des mitochondries lorsqu'il est induit par des agents capables de faire chuter le potentiel de membrane mitochondrial. Il est maintenant bien établi que le gonflement des mitochondries est provoqué par une modification de la perméabilité de la membrane interne des mitochondries à des petites molécules de poids moléculaire supérieur à 1500 daltons. Ce phénomène appelé perméabilité de transition consécutif à une chute de potentiel de la membrane est associé à l'ouverture irréversible d'un pore à haute conductance, un gonflement osmotique de la matrice et une libération de facteurs mitochondriaux ayant la capacité de déclencher les étapes initiales de l'apoptose (cytochrome c, facteur induisant l'apoptose) : voir Gunter, T.E. et Pfeiffer, D.R., Mechanisms by which mitochondria transport calcium, *Am. J. Physiol*. (1990), **258,** C755-C786 ; Hunter, D.R. et Haword, R.A., The Ca²⁺-induced membrane transition in mitochondria. III. Transitional Ca²⁺ release, *Arch. Biochem. Biophys.* (1979), **195,** 468-477; Bratton, S.B. et Cohen, G.M., Apoptotic death sensor: an organelle's alter ego, *TRENDS* (2001), **22,** 306-315).

Il devient alors particulièrement intéressant de trouver des composés qui empêcheraient ou diminueraient le gonflement des mitochondries en s'opposant à l'ouverture de ce pore à haute conductance. Cette propriété qui peut être démontrée sur des mitochondries isolées peut apporter des bénéfices cliniques dans des indications thérapeutiques différentes de celles décrites dans la demande de brevet PCT WO 01/26656, lesquelles consistent en des désordres génétiques ou fonctionnels mitochondriaux.

Le rapport entre le gonflement des mitochondries et certaines pathologies est notamment décrit dans les références suivantes :
- pour les maladies mitochondriales d'origine génétique : Clostre, Mitochondries : découvertes physiopathologiques récentes et nouvelles perspectives thérapeutiques, *Ann. Pharm. Fr.* (2001), **59**, 3-21 ;
- pour le sepsis (choc septique) : Fink, Cytopathic hypoxia. Mitochondrial dysfunction as mechanism contributing to organ dysfunction in sepsis, *Crit. Care Clin.* (2001), **17**, 219-237 ;
- pour la cirrhose du foie : Tsukamoto et coll., Current concept in the pathogenesis of alcoholic liver injury, FASEB J (2001), **15**(8) :1335-49 ;
- pour la toxicité cardiaque, rénale ou hépatique induite par des agents médicamenteux : Lewis et coll., Mitochondrial toxicity of antiviral drugs, *Nat. Med.,* **1**(5), 417-22.

Le fait que les composés de formule générale **(I)** décrite ci-après empêchent le gonflement des mitochondries permet donc d'envisager leur utilisation notamment pour préparer un médicament destiné à traiter une maladie / un désordre choisi parmi les maladies / désordres suivants les myopathies, les amyopathies, les ptosis, l'atrophie optique, la rétinite pigmentaire, la surdité, l'hépatomégalie, la cytolyse hépatique, la cardiomyopathie hypertrophique, l'ophtalmoplégie externe progressive chronique, le syndrome de Kearns Sayre, le syndrome de Leigh, le syndrome de Leber, le syndrome Narp, les syndromes MELAs, le syndrome de Pearson, le sepsis, la cirrhose du foie, et la toxicité cardiaque, rénale ou hépatique induite par des agents médicamenteux.

De préférence, les composés de formule générale **(I)** décrite ci-après seront utilisés pour préparer un médicament destiné à traiter une maladie / un désordre choisi parmi les maladies / désordres suivants : les amyopathies, les ptosis, l'atrophie optique, la rétinite pigmentaire, la surdité, la cytolyse hépatique, l'ophtalmoplégie externe progressive chronique, le syndrome de Kearns Sayre, le syndrome de Leigh, le syndrome de Leber, le syndrome Narp, les syndromes MELAs, le syndrome de Pearson, le sepsis, la cirrhose du foie, et la toxicité cardiaque, rénale ou hépatique induite par des agents médicamenteux.

Tout particulièrement, les composés de formule générale **(I)** décrite ci-après seront utilisés pour préparer un médicament destiné à traiter la cirrhose du foie.

Selon l'invention, les composés de formule générale **(I)** 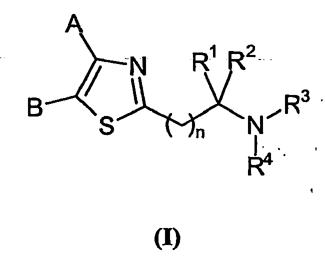 dans laquelle
A représente un radical **(A1)** 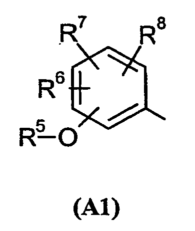
dans lequel R⁵ représente un atome d'hydrogène ou un radical alkyle, R⁶ représente un atome d'hydrogène ou un radical alkyle, cycloalkyle, hydroxy ou alkoxy, R⁷ représente un atome d'hydrogène ou un radical alkyle, cycloalkyle, hydroxy ou alkoxy et R⁸ représente un atome d'hydrogène ou un radical alkyle, cycloalkyle, hydroxy ou alkoxy,
ou encore A représente un radical **(A2)** 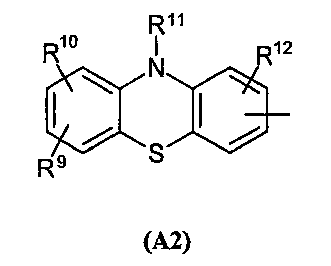 dans lequel R⁹ et R¹⁰ représentent indépendamment un atome d'hydrogène ou un radical hydroxy, alkyle ou alkoxy, R¹¹ représente un atome d'hydrogène ou un radical alkyle et R¹² représente un atome d'hydrogène ou un radical hydroxy, alkyle ou alkoxy ;
B représente un atome d'hydrogène ou un radical alkyle ;
n représente un entier de 0 à 5 ;
R¹ et R² représentent indépendamment un atome d'hydrogène ou un radical alkyle ou cycloalkyle ;
R³ et R⁴ représentent indépendamment un atome d'hydrogène ou un radical alkyle, ou bien R³ et R⁴ forment ensemble avec l'atome d'azote qui les porte un hétérocycle comptant en tout de 1 à 2 hétéroatomes et de 5 à 7 chaînons, hétérocycle dont les chaînons manquants sont choisis parmi -CHR¹³-, -NR¹⁴-, -O- et -S-, R¹³ représentant un atome d'hydrogène, le groupe -OH ou un radical alkyle ou alkoxy et R¹⁴ représentant un atome d'hydrogène ou un radical alkyle, -COR¹⁵, -COOR¹⁵ ou -CONR¹⁶R¹⁷, R¹⁵ représentant un radical alkyle et R¹⁶ et R¹⁷ représentent indépendamment un atome d'hydrogène ou un radical alkyle ;
ou les sels pharmaceutiquement acceptables des composés de formule générale **(I)**
peuvent être utilisés pour préparer un médicament destiné à protéger les mitochondries.

Par alkyle ou alkoxy, lorsqu'il n'est pas donné plus de précision, on entend un radical alkyle ou alkoxy linéaire ou ramifié comptant de 1 à 6 atomes de carbone. Par cycloalkyle, lorsqu'il n'est pas donné plus de précision, on entend un système monocyclique carboné comptant de 3 à 7 atomes de carbone. Enfin, par halogène, on entend les atomes de fluor, de chlore, de brome ou d'iode.

Par hétérocycle, on entend notamment les radicaux pipéridine, pipérazine, morpholine et thiomorpholine. Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt sélection for basic drugs", *Int. J. Pharm.* (1986), **33,** 201-217.

Par ailleurs, certains des composés de formule générale **(I)** peuvent se présenter sous la forme d'énantiomères. La présente invention inclut les deux formes énantiomères et toutes combinaisons de ces formes, y compris les mélanges racémiques "R,S". Dans un souci de simplicité, lorsqu'aucune configuration spécifique n'est indiquée dans les formules de structure, il faut comprendre que les deux formes énantiomères et leurs mélanges sont représentés.

De préférence, les composés de l'invention seront tels qu'ils comportent au moins l'une des caractéristiques suivantes :
- A représentant un radical **(A1)** 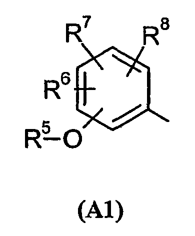 dans lequel R⁵ représente un atome d'hydrogène ou un radical méthyle, R⁶ représente un atome d'hydrogène ou un radical alkyle, cycloalkyle, hydroxy ou alkoxy, R⁷ représente un atome d'hydrogène ou un radical alkyle, cycloalkyle, hydroxy ou alkoxy et R⁸ représente un atome d'hydrogène ou un radical alkyle, cycloalkyle, hydroxy ou alkoxy,
   ou encore A représentant un radical **(A2)** 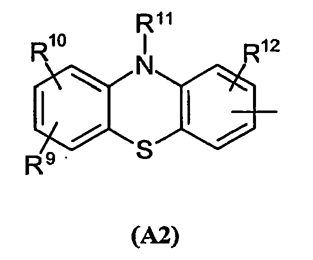 dans lequel R⁹ et R¹⁰ représentent indépendamment un atome d'hydrogène ou un radical hydroxy, alkyle ou alkoxy, R¹¹ représente un atome d'hydrogène ou un radical méthyle et R¹² représente un atome d'hydrogène ou un radical hydroxy, alkyle ou alkoxy ;
- B représentant un atome d'hydrogène ou un groupe méthyle ou éthyle ;
- n représentant un entier de 0 à 3 ;
- R¹ et R² représentant indépendamment un atome d'hydrogène ou un radical alkyle ;
- R³ et R⁴ représentant indépendamment un atome d'hydrogène ou un radical alkyle, ou bien R³ et R⁴ formant ensemble avec l'atome d'azote qui les porte un hétérocycle comptant en tout de 1 à 2 hétéroatomes et de 5 à 7 chaînons, hétérocycle dont les chaînons manquants sont choisis parmi -CHR¹³-, -NR¹⁴-, -O- et -S-, R¹³ représentant un atome d'hydrogène, le groupe -OH ou un radical méthyle ou méthoxy et R¹⁴ représentant un atome d'hydrogène ou un radical alkyle, -COR¹⁵, -COOR¹⁵ ou -CONR¹⁶R¹⁷, R¹⁵ représentant un radical alkyle et R¹⁶ et R¹⁷ représentent indépendamment un atome d'hydrogène ou un radical alkyle.

Plus préférentiellement, les composés de l'invention seront tels qu'ils comportent au moins l'une des caractéristiques suivantes :
- A représentant un radical **(A1)** 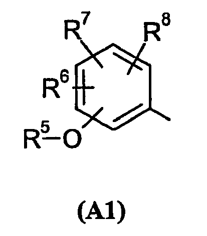 dans lequel R⁵ représente un atome d'hydrogène, R⁶ représente un atome d'hydrogène ou un radical alkyle, cycloalkyle, hydroxy ou alkoxy, R⁷ représente un atome d'hydrogène ou un radical alkyle, cycloalkyle, hydroxy ou alkoxy et R⁸ représente un atome d'hydrogène ou un radical alkyle, cycloalkyle, hydroxy ou alkoxy,
   ou encore A représentant un radical **(A2)** 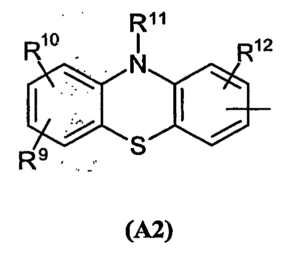 dans lequel R⁹ et R¹⁰ représentent indépendamment un atome d'hydrogène ou un radical hydroxy, alkyle ou alkoxy, R¹¹ représente un atome d'hydrogène et R¹² représente un atome d'hydrogène ou un radical hydroxy, alkyle ou alkoxy ;
- B représentant un atome d'hydrogène ou un groupe méthyle ;
- n représentant un entier de 0 à 2 ;
- l'un de R¹ et R² représentant un atome d'hydrogène, l'autre représentant un atome d'hydrogène ou un radical alkyle ou cycloalkyle ;
- R³ et R⁴ représentant indépendamment un atome d'hydrogène ou un radical alkyle, ou bien R³ et R⁴ formant ensemble avec l'atome d'azote qui les porte un hétérocycle à 6 chaînons comptant en tout de 1 à 2 hétéroatomes, hétérocycle dont les chaînons manquants sont choisis parmi -CHR¹³-, -NR¹⁴-, -O- et -S-, R¹³ représentant un atome d'hydrogène, le groupe -OH ou un radical méthyle et R¹⁴ représentant un atome d'hydrogène ou un radical alkyle.

Encore plus préférentiellement, les composés de l'invention seront tels qu'ils comportent au moins l'une des caractéristiques suivantes :
- A représentant un radical **(A1)** 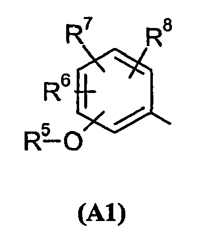 dans lequel R⁵ représente un atome d'hydrogène, R⁶ représente un atome d'hydrogène ou un radical alkyle, hydroxy ou alkoxy, R⁷ représente un atome d'hydrogène ou un radical alkyle, hydroxy ou alkoxy et R⁸ représente un atome d'hydrogène ou un radical alkyle, hydroxy ou alkoxy,
   ou encore A représentant un radical **(A2)** 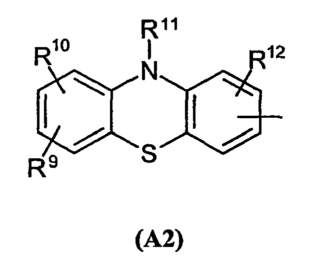 dans lequel R⁹ et R¹⁰ représentent indépendamment un atome d'hydrogène ou un radical hydroxy, méthyle, éthyle, méthoxy ou éthoxy, R¹¹ représente un atome d'hydrogène et R¹² représente un atome d'hydrogène ou un radical hydroxy, méthyle, éthyle, méthoxy ou éthoxy ;
- B représentant un atome d'hydrogène ;
- n représentant un entier de 0 à 1 ;
- l'un de R¹ et R² représentant un atome d'hydrogène, l'autre représentant un atome d'hydrogène ou un radical alkyle (et de préférence un radical alkyle linéaire ou ramifié comptant de 1 à 3 atomes de carbone, en particulier un radical méthyle ou éthyle) ;
- R³ et R⁴ représentant indépendamment un atome d'hydrogène ou un radical alkyle, ou bien R³ et R⁴ formant ensemble avec l'atome d'azote qui les porte un hétérocycle à 6 chaînons comptant en tout de 1 à 2 hétéroatomes, hétérocycle dont les chaînons manquants sont choisis parmi -CHR¹³-, -NR¹⁴-, -O- et -S-, R¹³ représentant un atome d'hydrogène, le groupe -OH ou un radical méthyle et R¹⁴ représentant un atome d'hydrogène ou un radical alkyle.

De façon particulièrement préférée, le radical **(A1)** sera un radical 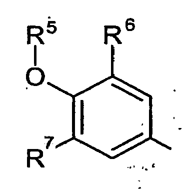 dans lequel R⁵ représente un atome d'hydrogène ou un radical alkyle (et en particulier un atome d'hydrogène), R⁶ représente un radical alkyle (en particulier le radical *iso*-propyle ou *tert*-butyle et plus particulièrement le radical *tert*-butyle) et R⁷ représente un radical alkyle (en particulier le radical *iso*-propyle ou *tert*-butyle et plus particulièrement le radical *tert*-butyle).

De même, de façon particulièrement préférée, le radical **(A2)** sera un radical 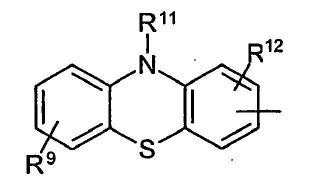 dans lequel R⁹ représente un atome d'hydrogène ou un radical méthyle ou méthoxy (et en particulier un atome d'hydrogène), R¹¹ représente un atome d'hydrogène ou un radical méthyle (et en particulier un atome d'hydrogène) et R¹² représente un atome d'hydrogène ou un radical méthyle ou méthoxy (et en particulier un atome d'hydrogène).

En outre :
- on préférera tout particulièrement les cas où n représente 0 ;
- la variante de l'invention selon laquelle A représente un radical **(A1)** sera généralement préférée par rapport à celle selon laquelle A représente un radical **(A2)** ;
- on préférera les cas où R³ et R⁴ représentent indépendamment un atome d'hydrogène ou un radical alkyle à ceux dans lesquels R³ et R⁴ forment ensemble avec l'atome d'azote qui les porte un hétérocycle ;
- lorsque R³ ou R⁴ représentera un radical alkyle, il s'agira de préférence d'un radical alkyle comptant de 1 à 3 atomes de carbone, et en particulier d'un radical méthyle ou éthyle (tout particulièrement d'un radical méthyle) ;
- lorsque R³ et R⁴ forment ensemble avec l'atome d'azote qui les porte un hétérocycle, cet hétérocycle sera de préférence un radical pipérazinyle, morpholinyle ou thiomorpholinyle (et plus préférentiellement un radical pipérazinyle) ou encore un radical pipéridinyle substitué (de préférence en position 3 ou 4) par un radical hydroxy ;
- lorsque R⁶, R⁷ ou R⁸ représentera un radical alkyle, il s'agira de préférence d'un radical alkyle comptant de 3 à 6 atomes de carbone, et en particulier d'un radical *tert*-butyle ou *iso*-propyle.

En particulier, les composés suivants peuvent être utilisés selon l'invention:
- 4-[3,5-bis(1,1-diméthyléthyl)-4-hydroxyphényl]-N-méthyl-1,3,thiazol-2-ylméthanamine ;
- N-méthyl[4-(10H-phénothiazin-2-yl)-1,3-thiazol-2-yl]méthanamine ;
- 2,6-di-tert butyl-4-{2-[(4-méthylpipérazin-1-yl)méthyl]-1,3-thiazol-4-yl}phénol ;
- 4-[2-(aminométhyl)-1,3-thiazol-4-yl]-2,6-di(*tert*-butyl)phénol ;
- 2,6-di-tert butyl-4-{2-[1-(méthylamino)éthyl]-1,3-thiazol-4-yl}phénol ;
ou leurs sels pharmaceutiquement acceptables.

Les compositions pharmaceutiques contenant un composé de l'invention peuvent être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'un produit selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

A titre indicatif, la dose d'administration envisagée pour un médicament selon l'invention est comprise entre 0,1 mg à 10 g suivant le type de composé actif utilisé.

La préparation des composés de formule générale **(I)** selon la présente invention est décrite dans la demande de brevet PCT WO 01/26656.

A moins qu'ils ne soient définis d'une autre manière, tous les termes techniques et scientifiques utilisés ici ont la même signification que celle couramment comprise par un spécialiste ordinaire du domaine auquel appartient cette invention.

### EXEMPLES

Les composés suivants :
- chlorhydrate de 4-[3,5-bis(1,1-diméthyléthyl)-4-hydroxyphényl]-N-méthyl-1,3-thiazol-2-yl méthanamine (composé 1),
- chlorhydrate de N-méthyl[4-(10H-phénothiazin-2-yl)-1,3-thiazol-2-yl]méthanamine (composé 2),
- chlorhydrate de 2,6-di-tert-butyl-4-{2-[(4-méthylpipérazin-1-yl)méthyl]-1,3-thiazol-4-yl}phénol (composé 3),
- chlorhydrate de 4-[2-(aminométhyl)-1,3-thiazol-4-yl]-2,6-di(*tert*-butyl)phénol (composé 4) et
- chlorhydrate de 2,6-di-tert-butyl-4-{2-[1-(méthylamino)éthyl]-1,3-thiazol-4-yl}phénol (composé 5)
ont été soumis au test d'étude du gonflement de mitochondries isolées de foie de rat comme décrit ci-après.

Tous les composés mentionnés ci-dessus ont été décrits dans la demande PCT WO 01/26656 ou sont accessibles par des méthodes de synthèse analogues à celles décrites dans ce document.

### Principe du test

Le test consiste à mesurer l'interaction des composés vis-à-vis du gonflement de mitochondries isolées de foie de rat, mesuré par absorbance spectophotométrique. Le gonflement des mitochondries isolées de foie est utilisé comme un indicateur de la modification de la perméabilité de transition et peut être induit par différents agents: le tert-butylhydroperoxyde (t-BH); l'ion méthyl phényl pyridinium (MPP⁺) et le dihydrogénophosphate de potassium (Pi) en présence de calcium (Ca²⁺).
Le t-BH est métabolisé par la gluthation peroxydase système, épuisant le pouvoir réducteur de la mitochondrie représenté par le NAD(P)H et le gluthation (GSH) et conduisant à l'oxydation des groupements SH.
Le MPP⁺ est un inhibiteur du complexe I de la chaîne de transport des électrons de la mitochondrie provoquant une génération de radicaux libres, une diminution du potentiel de membrane facilitant l'ouverture du pore de transition et l'extrusion du cytochrome c.

Le Pi déclenche la perméabilité de transition en diminuant la concentration d'ADP de la matrice, en stimulant la peroxydation lipidique et la production de radicaux libres mitochondriaux.

### Préparation des mitochondries de foie de rat

Le foie de rat Sprague-Dawley de 240-260 g, mis à jeun la veille, (Charles River, France) est prélevé, pesé, émincé dans 50 ml de tampon d'extraction (225 mM de mannitol; 75 mM de sucrose; 0,2 mM d'EDTA; 5 mM de TRIS-HCl, pH 7,4 à 4°C) et homogénéisé selon le protocole décrit par Johnson et Lardy (Isolation of liver and kidney mitochondria, *Methods Enzymol*. (1967), **10**, 94-96) et Holtzman et coll. (Effects of osmolar changes on isolated mitochondria of brain and liver, *J. Neurochem.* (1978), **30**, 1409-1419) à l'aide d'un homogénéiseur de verre (5 allers/retours). L'homogénat est centrifugé pendant 5 minutes à 1 085 g. Le surnageant résultant est centrifugé à 17 000 g. pendant 10 minutes. Puis le culot est repris dans 12,5 ml de tampon d'extraction par agitation douce à l'aide d'une tige de verre, puis la suspension est centrifugée à 17 000 g pendant 10 minutes. Le culot obtenu est remis en suspension dans 1 ml de tampon d'extraction à 4 °C. La concentration en protéines mitochondriales (68,48 ± 1,17 mg/ml) est déterminée par la méthode de Lowry (Protein measurement with the folin phenol reagent, *J Biol. Chem*, 93, 1951 : 265-275). La suspension mitochondriale est conservée dans la glace et utilisée dans les 3 heures.

### Mesure du gonflement des mitochondries de foie de rat

La quantification du gonflement des mitochondries est effectuée en mesurant à l'aide d'un spectrophotomètre (Shimadzu UV-2401PC) la dispersion de la lumière à 540 nm. Les mitochondries (concentration finale de 0,5 mg protéines/ml pour l'induction du gonflement par le Pi ou 1 mg/ml pour le t-BH et le MPP+) sont incubées dans 3,6 ml de tampon contenant :
- lorsque l'agent inducteur est t-BH : 225 mM de mannitol; 75 mM de sucrose, 3 mM d'HEPES, 5 mM de succinate, et 0,5 nmoles de roténone / mg de protéines, pH 7,4, à 25 °C ;
- lorsque l'agent inducteur est MPP+ : 225 mM de mannitol, 75 mM de sucrose, 5 mM de HEPES, 5 mM/0,5 mM de glutamate/malate, pH 7,4, à 25 °C ;
- lorsque l'agent inducteur est Pi : 150 mM de sucrose, 65 mM de KCl, 2,5 mM de succinate, 5 µM de roténone et 10 mM d'HEPES - KOH, pH 7,4, à 30 °C.

Un volume de 1,8 ml de la suspension correspondante est introduite dans la cuvette de mesure du spectrophotomètre ainsi que dans la cuvette dite de référence en présence des composés à tester. La mesure de variation d'absorbance (ΔA₅₄₀) des deux cuvettes est effectuée en parallèle.

### Induction du gonflement des mitochondries de foie de rat

Lorsque l'agent inducteur est t-BH : après 2 min d'incubation à 25 °C, 70 nmoles de CaCl₂ sont ajoutées et 2 minutes plus tard, 200 µM de t-BH sont introduits dans la cuvette de mesure [méthode modifiée de Broekemeir et Pfeiffer (Cyclosporin A is a potent inhibitor of the inner membrane permeability transition in liver mitochondria, *J. Biol. Chem*. (1989), **264,** 7826-7830)].
Lorsque l'agent inducteur est MPP+ : après 5 min d'incubation à 25 °C, 1 mM de MPP+ et 50 µM de Ca²⁺ sont introduits dans la cuvette de mesure suivis 2 min plus tard par 300 µM de Pi [méthode modifiée de Cassarino et coll. (The parkinsonian neurotoxin MPP+ opens the mitochondrial permeability transition pore and releases cytochrome c in isolated mitochondria via an oxidative mechanism, *Biochim. Biophys. Acta* (1999), **1453**, 49-62)].
Lorsque l'agent inducteur est Pi : après 1 min d'incubation à 30 °C, 10 µM de CaCl₂ sont introduits dans les deux cuvettes. Cinq minutes plus tard, le gonflement est déclenché par l'introduction de 4 mM de dihydrogénophosphate de potassium dans la cuvette de mesure uniquement [méthode modifiée de Kowaltowski et coll. (Effect of inorganic phosphate concentration on the nature of inner mitochondrial membrane alterations mediated by Ca²⁺ ions. A proposed model for phosphate-stimulated lipid peroxidation, *J. Biol. Chem.* (1996), **271,** 2929-2934) et Elimadi et coll. (Trimetazidine counteracts the hepatic injury associated with ischemia - reperfusion by preserving mitochondrial function, *J. Pharmacol. Exp. Ther.* (1998), **286,** 23-28)].

### Analyse des données

La vitesse de diminution de l'absorbance A₅₄₀ est proportionnelle à la vitesse de recrutement des mitochondries en phase de modification de perméabilité de transition. Cette vitesse est exprimée par le ΔA₅₄₀/min/mg protéine, calculée à partir de la tangente de la partie la plus pentue de l'absorbance en fonction du temps (UV-2101/3101PC Optional Kinetics Software). L'efficacité des produits, dont les effets sont testés deux à trois fois, est estimée par leur aptitude à diminuer de manière significative la vitesse de recrutement des mitochondries en phase de modification de perméabilité. Les comparaisons sont effectuées en utilisant une analyse de variance. Une valeur de *p* < 0,05 est considérée comme statistiquement significative.

### Résultats

Les composés 1 à 5 cités ci-dessus ont, à une concentration égale ou inférieure à 25 µM, significativement diminué la vitesse de recrutement des mitochondries en phase de modification de perméabilité induite soit par le tBH, le MPP+ ou le Pi.

## Revendications

1. Utilisation d'un composé de formule générale **(I)** 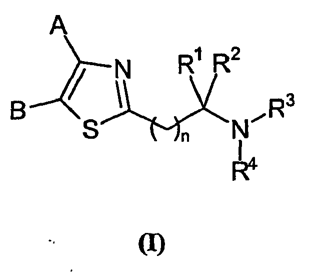 sous forme de mélange racémique, d'énantiomère ou de toute combinaison de ces formes, dans laquelle :
A représente un radical **(A1)** 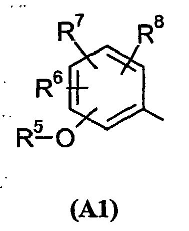
dans lequel R⁵ représente un atome d'hydrogène ou un radical alkyle, R⁶ représente un atome d'hydrogène ou un radical alkyle, cycloalkyle, hydroxy ou alkoxy, R⁷ représente un atome d'hydrogène ou un radical alkyle, cycloalkyle, hydroxy ou alkoxy et R⁸ représente un atome d'hydrogène ou un radical alkyle, cycloalkyle, hydroxy ou alkoxy,
ou encore A représente un radical **(A2)** 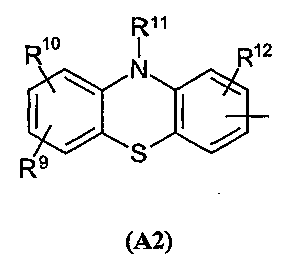 dans lequel R⁹ et R¹⁰ représentent indépendamment un atome d'hydrogène ou un radical hydroxy, alkyle ou alkoxy, R¹¹ représente un atome d'hydrogène ou un radical alkyle et R¹² représente un atome d'hydrogène ou un radical hydroxy, alkyle ou alkoxy ;
B représente un atome d'hydrogène ou un radical alkyle ;
n représente un entier de 0 à 5 ;
R¹ et R² représentent indépendamment un atome d'hydrogène ou un radical alkyle ou cycloalkyle ;
R³ et R⁴ représentent indépendamment un atome d'hydrogène ou un radical alkyle, ou bien R³ et R⁴ forment ensemble avec l'atome d'azote qui les porte un hétérocycle comptant en tout de 1 à 2 hétéroatomes et de 5 à 7 chaînons, hétérocycle dont les chaînons manquants sont choisis parmi -CHR¹³-, -NR¹⁴-, -O- et -S-, R¹³ représentant un atome d'hydrogène, le groupe -OH ou un radical alkyle ou alkoxy et R¹⁴ représentant un atome d'hydrogène ou un radical alkyle, -COR¹⁵, -COOR¹⁵ ou -CONR¹⁶R¹⁷, R¹⁵ représentant un radical alkyle et R¹⁶ et R¹⁷ représentent indépendamment un atome d'hydrogène ou un radical alkyle ;
étant entendu qu'un radical alkyle ou alkoxy, lorsqu'il n'est pas donné plus de précision, est linéaire ou ramifié et compte de 1 à 6 atomes de carbone et qu'un radical cycloalkyle, lorsqu'il n'est pas donné plus de précision, compte de 3 à 7 atomes de carbone ;
ou d'un sel pharmaceutiquement acceptable d'un composé de formule générale **(I)**
pour préparer un médicament destiné à traiter une maladie / un désordre choisi parmi les maladies / désordres suivants : les myopathies, les amyopathies, les ptosis, l'atrophie optique, la rétinite pigmentaire, la surdité, l'hépatomégalie, la cytolyse hépatique, la cardiomyopathie hypertrophique, l'ophtalmoplégie externe progressive chronique, le syndrome de Kearns Sayre, le syndrome de Leigh, le syndrome de Leber, le syndrome Narp, les syndromes MELAs, le syndrome de Pearson, le sepsis, la cirrhose du foie, et la toxicité cardiaque, rénale ou hépatique induite par des agents médicamenteux.

2. Utilisation selon la revendication 1, **caractérisée en ce que** :
• A représente un radical **(A1)** 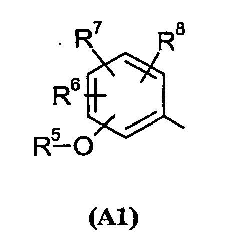 dans lequel R⁵ représente un atome d'hydrogène ou un radical méthyle, R⁶ représente un atome d'hydrogène ou un radical alkyle, cycloalkyle, hydroxy ou alkoxy, R⁷ représente un atome d'hydrogène ou un radical alkyle, cycloalkyle, hydroxy ou alkoxy et R⁸ représente un atome d'hydrogène ou un radical alkyle, cycloalkyle, hydroxy ou alkoxy,
ou encore A représentant un radical **(A2)** 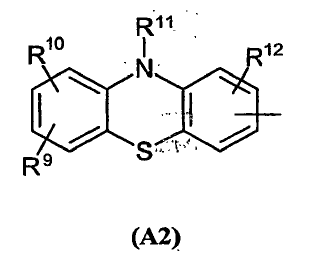 dans lequel R⁹ et R¹⁰ représentent indépendamment un atome d'hydrogène ou un radical hydroxy, alkyle ou alkoxy, R¹¹ représente un atome d'hydrogène ou un radical méthyle et R¹² représente un atome d'hydrogène ou un radical hydroxy, alkyle ou alkoxy ;
• B représente un atome d'hydrogène ou un groupe méthyle ou éthyle ;
• n représente un entier de 0 à 3 ;
• R¹ et R² représentent indépendamment un atome d'hydrogène ou un radical alkyle ;
• R³ et R⁴ représentent indépendamment un atome d'hydrogène ou un radical alkyle, ou bien R³ et R⁴ forment ensemble avec l'atome d'azote qui les porte un hétérocycle comptant en tout de 1 à 2 hétéroatomes et de 5 à 7 chaînons, hétérocycle dont les chaînons manquants sont choisis parmi -CHR¹³-, -NR¹⁴-, -O- et -S-, R¹³ représentant un atome d'hydrogène, le groupe -OH ou un radical méthyle ou méthoxy et R¹⁴ représentant un atome d'hydrogène ou un radical alkyle, -COR¹⁵, -COOR¹⁵ ou -CONR¹⁶R¹⁷, R¹⁵ représentant un radical alkyle et R¹⁶ et R¹⁷ représentent indépendamment un atome d'hydrogène ou un radical alkyle.

3. Utilisation selon la revendication 1, **caractérisée en ce que** :
• A représente un radical **(A1)** 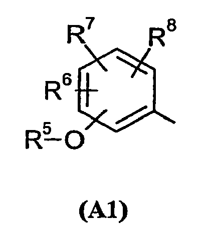 dans lequel R⁵ représente un atome d'hydrogène, R⁶ représente un atome d'hydrogène ou un radical alkyle, cycloalkyle, hydroxy ou alkoxy, R⁷ représente un atome d'hydrogène ou un radical alkyle, cycloalkyle, hydroxy ou alkoxy et R⁸ représente un atome d'hydrogène ou un radical alkyle, cycloalkyle, hydroxy ou alkoxy,
ou encore A représente un radical **(A2)** 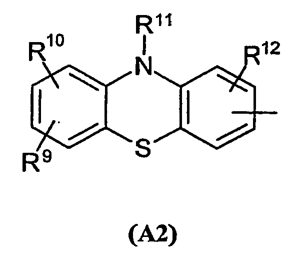 dans lequel R⁹ et R¹⁰ représentent indépendamment un atome d'hydrogène ou un radical hydroxy, alkyle ou alkoxy, R¹¹ représente un atome d'hydrogène et R¹² représente un atome d'hydrogène ou un radical hydroxy, alkyle ou alkoxy ;
• B représente un atome d'hydrogène ou un groupe méthyle ;
• n représente un entier de 0 à 2 ;
• l'un de R¹ et R² représente un atome d'hydrogène, l'autre représentant un atome d'hydrogène ou un radical alkyle ou cycloalkyle ;
• R³ et R⁴ représentent indépendamment un atome d'hydrogène ou un radical alkyle,
ou bien R³ et R⁴ forment ensemble avec l'atome d'azote qui les porte un hétérocycle à 6 chaînons comptant en tout de 1 à 2 hétéroatomes, hétérocycle dont les chaînons manquants sont choisis parmi -CHR¹³-, -NR¹⁴-, -O- et -S-, R¹³ représentant un atome d'hydrogène, le groupe -OH ou un radical méthyle et R¹⁴ représentant un atome d'hydrogène ou un radical alkyle.

4. Utilisation selon la revendication 1, **caractérisée en ce que** :
• A représente un radical **(A1)** 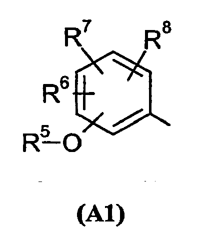 dans lequel R⁵ représente un atome d'hydrogène, R⁶ représente un atome d'hydrogène ou un radical alkyle, hydroxy ou alkoxy, R⁷ représente un atome d'hydrogène ou un radical alkyle, hydroxy ou akoxy et R⁸ représente un atome d'hydrogène ou un radical alkyle, hydroxy ou alkoxy,
ou encore A représente un radical **(A2)** 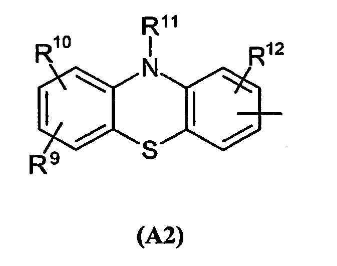 dans lequel R⁹ et R¹⁰ représentent indépendamment un atome d'hydrogène ou un radical hydroxy, méthyle, éthyle, méthoxy ou éthoxy, R¹¹ représente un atome d'hydrogène et R¹² représente un atome d'hydrogène ou un radical hydroxy, méthyle, éthyle, méthoxy ou éthoxy ;
• B représente un atome d'hydrogène ;
• n représente un entier de 0 à 1 ;
• l'un de R¹ et R² représente un atome d'hydrogène, l'autre représentant un atome d'hydrogène ou un radical alkyle (et de préférence un radical alkyle linéaire ou ramifié comptant de 1 à 3 atomes de carbone, en particulier un radical méthyle ou éthyle) ;
• R³ et R⁴ représentent indépendamment un atome d'hydrogène ou un radical alkyle,
ou bien R³ et R⁴ forment ensemble avec l'atome d'azote qui les porte un hétérocycle à 6 chaînons comptant en tout de 1 à 2 hétéroatomes, hétérocycle dont les chaînons manquants sont choisis parmi -CHR¹³-, -NR¹⁴-, -O- et -S-, R¹³ représentant un atome d'hydrogène, le groupe -OH ou un radical méthyle et R¹⁴ représentant un atome d'hydrogène ou un radical alkyle.

5. Utilisation selon la revendication 1, **caractérisée en ce que** n représente 0.

6. Utilisation selon l'une des revendications 1 à 5, **caractérisée en ce que** A représente un radical **(A1).**

7. Utilisation selon la revendication 1, **caractérisée en ce que** l'un des composés suivants est utilisé :
- 4-[3,5-bis(1,1-diméthyléthyl)-4-hydioxyphényl]-N-méthyl-1,3 thiazol-2-ylméthanamine ;
- N-méthyl[4-(10H-phénothiazin-2-yl)-1,3-thiazol-2-yl]méthanamine;
- 2,6-di-tert-butyl-4-{2-[(4-méthylpipérazin-1-yl)méthyl]-1,3-thiazol-4-yl}phénol ;
- 4-[2-(aminométhyl)-1,3-thiazol-4-yl]-2,6-di(*tert*-butyl)phénol ;
- 2,6-di-tert-butyl-4-{2-[1-(méthylamino)éthyl]-1,3-thiazol-4-yl}phénol ;
ou un sel pharmaceutiquement acceptable d'un de ces derniers.

8. Utilisation selon l'une des revendications 1 à 7, **caractérisée en ce que** le médicament préparé est destiné à traiter une maladie / un désordre choisi parmi les maladies / désordres suivants : les amyopathies, les ptosis, l'atrophie optique, la rétinite pigmentaire, la surdité, la cytolyse hépatique, l'ophtalmoplégie externe progressive chronique, le syndrome de Kearns Sayre, le syndrome de Leigh, le syndrome de Leber, le syndrome Narp, les syndromes MELAs, le syndrome de Pearson, le sepsis, la cirrhose du foie, et la toxicité cardiaque, rénale ou hépatique induite par des agents médicamenteux.

9. Utilisation selon la revendication 8, **caractérisée en ce que** le médicament préparé est destiné à traiter la cirrhose du foie.

## Claims

1. Use of a compound of general formula **(I)** 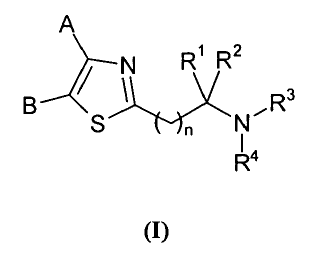 in the form of a racemic mixture, of an enantiomer or of any combination of these forms, in which:
A represents an **(A1)** radical 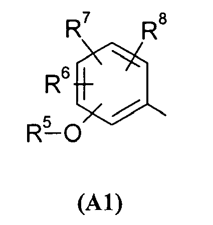
in which R⁵ represents a hydrogen atom or an alkyl radical, R⁶ represents a hydrogen atom or an alkyl, cycloalkyl, hydroxy or alkoxy radical, R⁷ represents a hydrogen atom or an alkyl, cycloalkyl, hydroxy or alkoxy radical and R⁸ represents a hydrogen atom or an alkyl, cycloalkyl, hydroxy or alkoxy radical,
or also A represents an **(A2)** radical 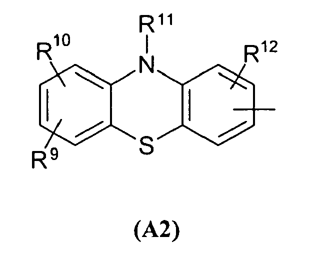 in which R⁹ and R¹⁰ represent independently a hydrogen atom or a hydroxy, alkyl or alkoxy radical, R¹¹ represents a hydrogen atom or an alkyl radical and R¹² represents a hydrogen atom or a hydroxy, alkyl or alkoxy radical;
B represents a hydrogen atom or an alkyl radical;
n represents an integer from 0 to 5;
R¹ and R² represent independently a hydrogen atom or an alkyl or cycloalkyl radical;
R³ and R⁴ represent independently a hydrogen atom or an alkyl radical, or R³ and R⁴ form together with the nitrogen atom which carries them a heterocycle containing in total 1 to 2 heteroatoms and 5 to 7 members, the missing members of which heterocycle are chosen from -CHR¹³-, -NR¹⁴-, -O- and -S-, R¹³ representing a hydrogen atom, the -OH group or an alkyl or alkoxy radical and R¹⁴ representing a hydrogen atom or an alkyl, -COR¹⁵, -COOR¹⁵ or -CONR¹⁶R¹⁷ radical, R¹⁵ representing an alkyl radical and R¹⁶ and R¹⁷ represent independently a hydrogen atom or an alkyl radical;
it being understood that an alkoxy or alkyl radical, unless specified otherwise, is linear or branched and contains from 1 to 6 carbon atoms and that a cycloalkyl radical, unless specified otherwise, contains from 3 to 7 carbon atoms;
or a pharmaceutically acceptable salt of a compound of general formula **(I)**
for preparing a medicament intended to treat a disease / a disorder chosen from the following diseases / disorders: myopathies, amyopathies, ptosis, optical atrophy, pigmentary retinitis, deafness, hepatomegalia, hepatic cytolysis, hypertrophic cardiomyopathy, chronic progressive external ophthalmoplegia, Kearns-Sayre syndrome, Leigh's syndrome, Leber's syndrome, NARP syndrome, MELAS syndromes, Pearson's syndrome, sepsis, cirrhosis of the liver, and cardiac, renal or hepatic toxicity induced by medicamentous agents.

2. Use according to claim 1, **characterized in that**:
• A represents an **(A1)** radical 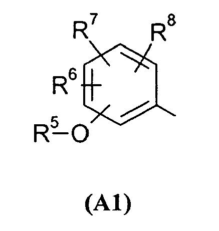 in which R⁵ represents a hydrogen atom or a methyl radical, R⁶ represents a hydrogen atom or an alkyl, cycloalkyl, hydroxy or alkoxy radical, R⁷ represents a hydrogen atom or an alkyl, cycloalkyl, hydroxy or alkoxy radical and R⁸ represents a hydrogen atom or an alkyl, cycloalkyl, hydroxy or alkoxy radical,
or also A representing an **(A2)** radical 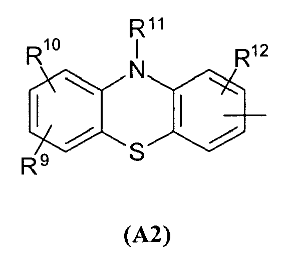 in which R⁹ and R¹⁰ represent independently a hydrogen atom or a hydroxy, alkyl or alkoxy radical, R¹¹ represents a hydrogen atom or a methyl radical and R¹² represents a hydrogen atom or a hydroxy, alkyl or alkoxy radical;
• B represents a hydrogen atom or a methyl or ethyl group;
• n represents an integer from 0 to 3;
• R¹ and R² represent independently a hydrogen atom or an alkyl radical;
• R³ and R⁴ represent independently a hydrogen atom or an alkyl radical, or R³ and R⁴ form together with the nitrogen atom which carries them a heterocycle containing in total 1 to 2 heteroatoms and 5 to 7 members, the missing members of which heterocycle are chosen from -CHR¹³-, -NR¹⁴-, -O- and -S-, R¹³ representing a hydrogen atom, the -OH group or a methyl or methoxy radical and R¹⁴ representing a hydrogen atom or an alkyl, -COR¹⁵, -COOR¹⁵ or -CONR¹⁶R¹⁷ radical, R¹⁵ representing an alkyl radical and R¹⁶ and R¹⁷ represent independently a hydrogen atom or an alkyl radical.

3. Use according to claim 1, **characterized in that**:
• A represents an **(A1)** radical 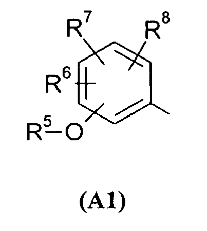 in which R⁵ represents a hydrogen atom, R⁶ represents a hydrogen atom or an alkyl, cycloalkyl, hydroxy or alkoxy radical, R⁷ represents a hydrogen atom or an alkyl, cycloalkyl, hydroxy or alkoxy radical and R⁸ represents a hydrogen atom or an alkyl, cycloalkyl, hydroxy or alkoxy radical,
or also A represents an **(A2)** radical 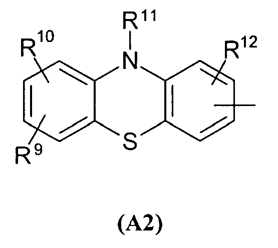 in which R⁹ and R¹⁰ represent independently a hydrogen atom or a hydroxy, alkyl or alkoxy radical, R¹¹ represents a hydrogen atom and R¹² represents a hydrogen atom or a hydroxy, alkyl or alkoxy radical;
• B represents a hydrogen atom or a methyl group;
• n represents an integer from 0 to 2;
• one of R¹ and R² represents a hydrogen atom, the other representing a hydrogen atom or an alkyl or cycloalkyl radical;
• R³ and R⁴ represent independently a hydrogen atom or an alkyl radical, or R³ and R⁴ form together with the nitrogen atom which carries them a heterocycle with 6 members containing in total 1 to 2 heteroatoms, the missing members of which heterocycle are chosen from -CHR¹³-, -NR¹⁴-, -O- and -S-, R¹³ representing a hydrogen atom, the -OH group or a methyl radical and R¹⁴ representing a hydrogen atom or an alkyl radical.

4. Use according to claim 1, **characterized in that**:
• A represents an **(A1)** radical 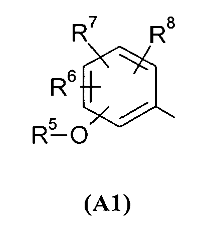 in which R⁵ represents a hydrogen atom, R⁶ represents a hydrogen atom or an alkyl, hydroxy or alkoxy radical, R⁷ represents a hydrogen atom or an alkyl, hydroxy or alkoxy radical and R⁸ represents a hydrogen atom or an alkyl, hydroxy or alkoxy radical,
or also A represents an **(A2)** radical 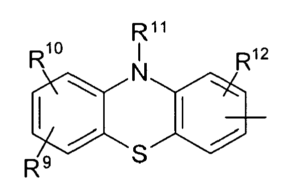 in which R⁹ and R¹⁰ represent independently a hydrogen atom or a hydroxy, methyl, ethyl, methoxy or ethoxy radical, R¹¹ represents a hydrogen atom and R¹² represents a hydrogen atom or a hydroxy, methyl, ethyl, methoxy or ethoxy radical;
• B represents a hydrogen atom;
• n represents an integer from 0 to 1;
• one of R¹ and R² represents a hydrogen atom, the other representing a hydrogen atom or an alkyl radical (and preferably a linear or branched alkyl radical containing 1 to 3 carbon atoms, in particular a methyl or ethyl radical);
• R³ and R⁴ represent independently a hydrogen atom or an alkyl radical, or R³ and R⁴ form together with the nitrogen atom which carries them a heterocycle with 6 members containing in total 1 to 2 heteroatoms, the missing members of which heterocycle are chosen from -CHR¹³-, -NR¹⁴-, -O- and -S-, R¹³ representing a hydrogen atom, the -OH group or a methyl radical and R¹⁴ representing a hydrogen atom or an alkyl radical.

5. Use according to claim 1, **characterized in that** n represents 0.

6. Use according to one of claims 1 to 5, **characterized in that** A represents an **(A1)** radical.

7. Use according to claim 1, **characterized in that** one of the following compounds is used:
- 4-[3,5-bis(1,1-dimethylethyl)-4-hydroxyphenyl]-N-methyl-1,3-thiazol-2-yl;
- N-methyl[4-(10H-phenothiazin-2-yl)-1,3-thiazol-2-yl]methanamine;
- 2,6-di-*tert*-butyl-4-{2-[(4-methylpiperazin-1-yl)methyl]-1,3-thiazol-4-yl}phenol;
- 4-[2-(aminomethyl)-1,3-thiazol-4-yl]-2,6-di(*tert*-butyl)phenol;
- 2,6-di-*tert*-butyl-4-{2-[1-(methylamino)ethyl]-1,3-thiazol-4-yl}phenol;
or a pharmaceutically acceptable salt of one of the latter.

8. Use according to one of claims 1 to 7, **characterized in that** the medicament prepared is intended to treat a disease / a disorder chosen from the following diseases / disorders: amyopathies, ptosis, optical atrophy, pigmentary retinitis, deafness, hepatic cytolysis, chronic progressive external ophthalmoplegia, Kearns-Sayre syndrome, Leigh's syndrome, Leber's syndrome, NARP syndrome, MELAS syndromes, Pearson's syndrome, sepsis, cirrhosis of the liver, and cardiac, renal or hepatic toxicity induced by medicamentous agents.

9. Use according to claim 8, **characterized in that** the medicament prepared is intended to treat cirrhosis of the liver.

## Patentansprüche

1. Verwendung einer Verbindung der allgemeinen Formel (I) 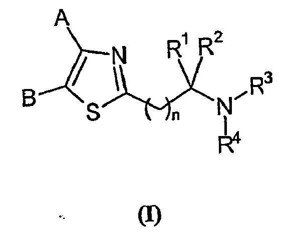 in Form von racemischer Mischung, Enantiomer oder jeder Kombination dieser Formen, in der:
A einen Rest (A1) darstellt, 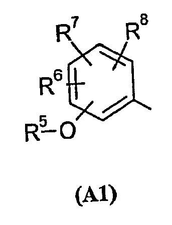
in dem R⁵ ein Wasserstoffatom oder einen Alkylrest darstellt, R⁶ ein Wasserstoffatom oder einen Alkyl-, Cycloalkyl-, Hydroxy- oder Alkoxyrest darstellt, R⁷ ein Wasserstoffatom oder einen Alkyl-, Cycloalkyl-, Hydroxy- oder Alkoxyrest darstellt und R⁸ ein Wasserstoffatom oder einen Alkyl-, Cycloalkyl-, Hydroxy- oder Alkoxyrest darstellt,
oder A einen Rest (A2) darstellt, 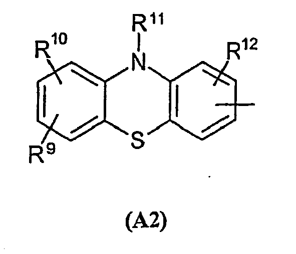 in dem R⁹ und R¹⁰ unabhängig voneinander ein Wasserstoffatom oder einen Hydroxy-, Alkyl- oder Alkoxyrest darstellen, R¹¹ ein Wasserstoffatom oder einen Alkylrest darstellt und R¹² ein Wasserstoffatom oder einen Hydroxy-, Alkyl- oder Alkoxyrest darstellt;
B ein Wasserstoffatom oder einen Alkylrest darstellt;
n eine ganze Zahl von 0 bis 5 darstellt;
R¹ und R² unabhängig voneinander ein Wasserstoffatom oder einen Alkyl- oder Cycloalkylrest darstellen;
R³ und R⁴ unabhängig voneinander ein Wasserstoffatom oder einen Alkylrest darstellen oder R³ und R⁴ zusammen mit dem sie tragenden Stickstoffatom einen Heterocyclus mit insgesamt 1 bis 2 Heteroatomen und 5 bis 7 Gliedern bilden, dessen fehlende Glieder aus -CHR¹³-, -NR¹⁴-, -O- und -S- ausgewählt sind, wobei R¹³ ein Wasserstoffatom, die -OH-Gruppe oder einen Alkyl- oder Alkoxyrest darstellt und R¹⁴ ein Wasserstoffatom oder einen der Reste Alkyl-, -COR¹⁵, -COOR¹⁵ oder -CONR¹⁶R¹⁷ darstellt, wobei R¹⁵ einen Alkylrest darstellt, und R¹⁶ und R¹⁷ unabhängig voneinander ein Wasserstoffatom oder einen Alkylrest darstellen;
wobei gilt, dass ein Alkyl- oder Alkoxyrest, wenn nicht Genaueres angegeben ist, linear oder verzweigt ist und 1 bis 6 Kohlenstoffatome zählt und dass ein Cycloalkylrest, wenn nicht Genaueres angegeben ist, 3 bis 7 Kohlenstoffatome zählt;
oder ein pharmazeutisch annehmbares Salz einer Verbindung der allgemeinen Formel (I)
zur Herstellung eines Medikaments, das zur Behandlung einer Krankheit/Störung bestimmt ist, die aus den folgenden Krankheiten/Störungen ausgewählt ist: Myopathien, Amyopathien, Ptosis, optische Atrophie, Retinitis pigmentosa, Schwerhörigkeit, Hepatomegalie, Leberzytolyse, hypertrophische Kardiomyopathie, Ophtalmoplegia chronica progressiva externa, Kearns-Sayre-Syndrom, Leigh-Syndrom, Leber-Syndrom, Narp-Syndrom, MELAS-Syndrome, Pearson-Syndrom, Sepsis, Leberzirrhose, durch medikamentöse Mittel induzierte Herz-, Nieren- oder Lebertoxizität.

2. Verwendung nach Anspruch 1, **dadurch gekennzeichnet, dass**
• A einen Rest (A1) darstellt, 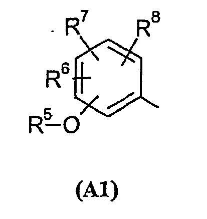 in dem R⁵ ein Wasserstoffatom oder einen Methylrest darstellt, R⁶ ein Wasserstoffatom oder einen Alkyl-, Cycloalkyl-, Hydroxy- oder Alkoxyrest darstellt, R⁷ ein Wasserstoffatom oder einen Alkyl-, Cycloalkyl-, Hydroxy- oder Alkoxyrest darstellt und R⁸ ein Wasserstoffatom oder einen Alkyl-, Cycloalkyl-, Hydroxy- oder Alkoxyrest darstellt,
oder A einen Rest (A2) darstellt, 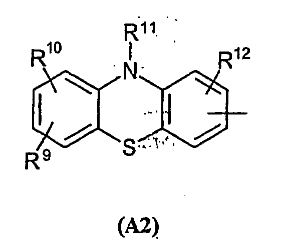 in dem R⁹ und R¹⁰ unabhängig voneinander ein Wasserstoffatom oder einen Hydroxy-, Alkyl- oder Alkoxyrest darstellen, R¹¹ ein Wasserstoffatom oder einen Methylrest darstellt und R¹² ein Wasserstoffatom oder einen Hydroxy-, Alkyl- oder Alkoxyrest darstellt;
• B ein Wasserstoffatom oder einen Methyl- oder Ethylgruppe darstellt;
• n eine ganze Zahl von 0 bis 3 darstellt;
• R¹ und R² unabhängig voneinander ein Wasserstoffatom oder einen Alkylrest darstellen;
• R³ und R⁴ unabhängig voneinander ein Wasserstoffatom oder einen Alkylrest darstellen oder R³ und R⁴ zusammen mit dem sie tragenden Stickstoffatom einen Heterocyclus mit insgesamt 1 bis 2 Heteroatomen und 5 bis 7 Gliedern bilden, dessen fehlende Glieder aus -CHR¹³-, -NR¹⁴-, -O- und -S- ausgewählt sind, wobei R¹³ ein Wasserstoffatom, die -OH-Gruppe oder einen Methyl- oder Methoxyrest darstellt und R¹⁴ ein Wasserstoffatom oder einen der Reste Alkyl, -COR¹⁵, -COOR¹⁵ oder -CONR¹⁶R¹⁷ darstellt, wobei R¹⁵ einen Alkylrest darstellt und R¹⁶ und R¹⁷ unabhängig voneinander ein Wasserstoffatom oder einen Alkylrest darstellen.

3. Verwendung nach Anspruch 1, **dadurch gekennzeichnet, dass**
• A einen Rest (A1) darstellt, 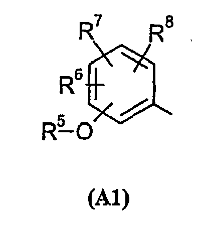 in dem R⁵ ein Wasserstoffatom darstellt, R⁶ ein Wasserstoffatom oder einen Alkyl-, Cycloalkyl-, Hydroxy- oder Alkoxyrest darstellt, R⁷ ein Wasserstoffatom oder einen Alkyl-, Cycloalkyl-, Hydroxy- oder Alkoxyrest darstellt und R⁸ ein Wasserstoffatom oder einen Alkyl-, Cycloalkyl-, Hydroxy- oder Alkoxyrest darstellt,
oder A einen Rest (A2) darstellt, 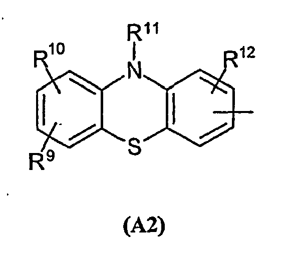 in dem R⁹ und R¹⁰ unabhängig voneinander ein Wasserstoffatom oder einen Hydroxy-, Alkyl- oder Alkoxyrest darstellen, R¹¹ ein Wasserstoffatom darstellt und R¹² ein Wasserstoffatom oder einen Hydroxy-, Alkyl- oder Alkoxyrest darstellt;
• B ein Wasserstoffatom oder eine Methylgruppe darstellt;
• n eine ganze Zahl von 0 bis 2 darstellt;
• einer der Reste R¹ und R² ein Wasserstoffatom darstellt, wobei der andere ein Wasserstoffatom oder einen Alkyl- oder Cycloalkylrest darstellt;
• R³ und R⁴ unabhängig voneinander ein Wasserstoffatom oder einen Alkylrest darstellen oder R³ und R⁴ zusammen mit dem sie tragenden Stickstoffatom einen Heterocyclus mit 6 Gliedern bilden, der insgesamt 1 bis 2 Heteroatome zählt und dessen fehlende Glieder aus -CHR¹³-, -NR¹⁴-, -O- und -S- ausgewählt sind, wobei R¹³ ein Wasserstoffatom, die -OH-Gruppe oder einen Methylrest darstellt und R¹⁴ ein Wasserstoffatom oder einen Alkylrest darstellt.

4. Verwendung nach Anspruch 1, **dadurch gekennzeichnet, dass**
• A einen Rest (A1) darstellt, 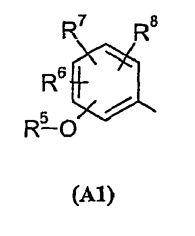 in dem R⁵ ein Wasserstoffatom darstellt, R⁶ ein Wasserstoffatom oder einen Alkyl-, Hydroxy- oder Alkoxyrest darstellt, R⁷ ein Wasserstoffatom oder einen Alkyl-, Hydroxy- oder Alkoxyrest darstellt, R8 ein Wasserstoffatom oder einen Alkyl-, Hydroxy- oder Alkoxyrest darstellt,
oder A einen Rest (A2) darstellt, 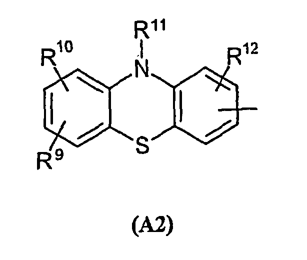 in dem R⁹ und R¹⁰ unabhängig voneinander ein Wasserstoffatom oder einen Hydroxy-, Methyl-, Ethyl-, Methoxy- oder Ethoxyrest darstellen, R¹¹ ein Wasserstoffatom darstellt und R¹² ein Wasserstoffatom oder einen Hydroxy-, Methyl-, Ethyl-, Methoxy- oder Ethoxyrest darstellt;
• B ein Wasserstoffatom darstellt;
• n eine ganze Zahl von 0 bis 1 darstellt;
• einer der Reste R¹ und R² ein Wasserstoffatom darstellt, wobei der andere ein Wasserstoffatom oder einen Alkylrest darstellt (und vorzugsweise einen linearen oder verzweigten Alkylrest mit 1 bis 3 Kohlenstoffatomen, insbesondere einen Methyl- oder Ethylrest);
• R³ und R⁴ unabhängig voneinander ein Wasserstoffatom oder einen Alkylrest darstellen oder R³ und R⁴ zusammen mit dem sie tragenden Stickstoffatom einen Heterocyclus mit 6 Gliedern bilden, der insgesamt 1 bis 2 Heteroatome zählt und dessen fehlende Glieder aus -CHR¹³-, -NR¹⁴-, -O- und -S- ausgewählt sind, wobei R¹³ ein Wasserstoffatom, die -OH-Gruppe oder einen Methylrest darstellt und R¹⁴ ein Wasserstoffatom oder einen Alkylrest darstellt.

5. Verwendung nach Anspruch 1, **dadurch gekennzeichnet, dass** n 0 darstellt.

6. Verwendung nach einem der Ansprüche 1 bis 5, **dadurch gekennzeichnet, dass** A einen Rest (A1) darstellt.

7. Verwendung nach Anspruch 1, **dadurch gekennzeichnet, dass** eine der folgenden Verbindungen verwendet wird:
- 4-[3,5-bis(1,1-Dimethylethyl)-4-hydroxyphenyl]-N-methyl-1,3-thiazol-2-yl;
- N-Methyl[4-(10H-phenothiazin-2-yl)-1,3-thiazol-2-yl]methanamin;
- 2,6-di-tert-Butyl-4-{2-[(4-methylpiperazin-1-yl)methyl]-1,3-thiazol-4-yl}phenol;
- 4-[2-(Aminomethyl)-1,3-thiazol-4-yl]-2,6-di(tert-butyl)phenol;
- 2,6-di-tert-Butyl-4-{2-[1-(methylamino)ethyl]-1,3-thiazol-4-yl}phenol;
oder ein pharmazeutisch annehmbares Salz dieser Verbindungen.

8. Verwendung nach einem der Ansprüche 1 bis 7, **dadurch gekennzeichnet, dass** das hergestellte Medikament für die Behandlung einer Krankheit/Störung bestimmt ist, die aus den folgenden Krankheiten/Störungen ausgewählt ist: Amyopathien, Ptosis, optische Atrophie, Retinitis pigmentosa, Schwerhörigkeit, Leberzytolyse, Ophtalmoplegia chronica progressiva externa, Kearns-Sayre-Syndrom, Leigh-Syndrom, Leber-Syndrom, Narp-Syndrom, MELAS-Syndrome, Pearson-Syndrom, Sepsis, Leberzirrhose, durch medikamentöse Mittel induzierte Herz-, Nieren- oder Lebertoxizität.

9. Verwendung nach Anspruch 8, **dadurch gekennzeichnet, dass** das hergestellte Medikament für die Behandlung der Leberzirrhose bestimmt ist.
